(19) **Europäisches Patentamt / European Patent Office / Office européen des brevets**

(11) **EP 1 847 294 B1**

(12) **EUROPEAN PATENT SPECIFICATION**

(45) Date of publication and mention
of the grant of the patent:
**14.10.2009 Bulletin 2009/42**

(51) Int Cl.:
***A61N 7/00*** (2006.01)

(21) Application number: **05781862.7**

(86) International application number:
**PCT/CN2005/001328**

(22) Date of filing: **26.08.2005**

(87) International publication number:
**WO 2006/079265 (03.08.2006 Gazette 2006/31)**

(54) **FOCUSED ULTRASOUND THERAPY SYSTEM**

THERAPIESYSTEM MIT FOKUSSIERTEM ULTRASCHALL

SYSTEME THERAPEUTIQUE ULTRASONORE FOCALISE

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.01.2005 CN 200510004980**

(43) Date of publication of application:
**24.10.2007 Bulletin 2007/43**

(73) Proprietor: **CHONGQING HAIFU(HIFU) TECHNOLOGY CO., LTD**
**Chongqing 401121 (CN)**

(72) Inventors:
• **CHEN, Wenzhi**
**Yubei District, Chongqing 401121 (CN)**
• **WEN, Yingang**
**Yubei District, Chongqing 401121 (CN)**

• **Wang, Zhibao**
**Yubei District, Chongqing 401121 (CN)**
• **WANG, Zhilong**
**Yubei District, Chongqing 401121 (CN)**
• **LIN, Tao**
**Yubei District, Chongqing 401121 (CN)**

(74) Representative: **Kampfenkel, Klaus**
**Blumbach - Zinngrebe**
**Patentanwälte**
**Alexandrastrasse 5**
**65187 Wiesbaden (DE)**

(56) References cited:
WO-A-20/04042546     WO-A1-02/09588
US-A- 4 932 414        US-B1- 6 540 700
US-B1- 6 685 639       US-B1- 6 725 481

## Description

### FIELD OF THE INVENTION

The present invention pertains to an ultrasound therapy system and more specifically, to a high-intensity focused ultrasound (HIFU) therapy apparatus guided by using image registration and fusion method.

### BACKGROUND OF THE INVENTION

In recent years, the using scope of the ultrasound therapy has become wider increasingly. With the development of ultrasound therapy, the special ultrasound therapy, in particular, the High-intensity focused ultrasound (HIFU) applies a damaging dose to effectively treat many kinds of diseases, particularly the tumor. Comparing to the conventional surgery and chemotherapy, HIFU therapy brings the patient less trauma and can realize a non-invasive treatment. Therefore its clinical applications have been developed rapidly. Its indications include liver cancer, bone sarcoma, breast cancer, pancreas cancer, kidney cancer, soft tissue tumor, pelvic tumor and etc.

The ultrasound tumor therapy devices normally adopt the sphere focusing. The ultrasound waves emitted from every point are directed to the center of sphere and become the focused ultrasound. The emitter on the ultrasound therapy device emits the ultrasound waves from outside of the body to inside of the body, which are focused during emission and transmission to form a high-energy focal point. Accordingly high-intensity and continuous ultrasound energy is applied to the target region of the subject. The transient high temperature effects (65~100°C), cavitation effects, mechanical effects and acoustic-chemical effects produced at the focal point are used to selectively cause coagulative necrosis of the diseased tissue and disable the proliferation, invasion and metastasis of tumor.

During the application of high-intensity focused ultrasound therapy, the accurate, safe and effective localization of the focal point is essential for a successful treatment and also the convenience of operations for locating the target needs to be improved further.

The medical imaging has become a necessary part of modern medical treatment. Its applications are run through the whole clinical work. Not only it has been used widely for diagnosing diseases, but also it has played an important role in planning surgical procedures, implementing the protocols and evaluating therapeutic effects of surgery and radiotherapy. At present, the medical images can be divided into two categories, such as anatomical images and functional images. Anatomical images mainly describes the human morphological information, including the serial images gained by X-ray imaging, CT, MRI, US and all kinds of endoscopic mirrors (for example, belly cavity mirror and laryngoscope). In addition, also some derivative special technologies, for example, DSA derived from X-ray imaging, MRA derived from MRI and Doppler Imaging derived from US imaging. Functional images mainly describes the human metabolic information, including PET, SPECT, fMRI and etc. Meanwhile, also there are some functional imaging methods in a broad sense or less used, for example, EEG, MEG, pMRI (perfusion MRI), fCT and etc.

With vigorous developments of Medical Imageology, it provides many kinds of useful information for diagnosis to ultrasound therapy and more options for localization of treatment. In the field of ultrasound therapy, normally B-mode ultrasound imaging device (B-mode scanner) is adopted, and X-ray computed tomography scanner (CT) and Magnetic Resonance Imaging (MRI) are also used. B-mode scanner, CT and MRI are respectively briefed thereinafter.

B-mode ultrasound imaging

The existing focused ultrasound therapy systems generally adopt B-mode Scanner to locate and monitor the treatment. The same inventors of the present invention submitted the application with a title "high intensity focused ultrasound System for scanning and curing tumor" on Jan. 25, 1998 and this Chinese Patent No. 98100283.8 was authorized and declared on Nov. 29, 2000. In this patent, this technical solution has been disclosed in detail. The full text is used as a reference here. A system of therapeutic ultrasound and real-time ultrasonic scanning is described in the document US 4,932,414. B-mode ultrasound monitoring system has the following advantages: low cost, real time imaging, having the same acoustic path as the therapeutic ultrasound, observing the tissue necrosis after high-intensity focused ultrasound (HIFU) exposures according to gray scale changes of the images. But, the ultrasound image is limited on the depth of observation and it almost cannot display the tissue behind the bone because the bone influences the image greatly, and the serious noises on the images exist during monitoring treatment. Further, the ultrasound image has poor capacity to identify the tissue boundary and its resolution for tumors is not ideal, particularly B-mode ultrasound image almost can not identify the small tumors and deep-bedded tumors, therefore, it is very difficult for an operator to determine the boundary of tumor and sometimes the tumor even completely can not be determined. Under this case, some operators approximately determine the tumor according to the relationship between the tumor and the surrounding tissue in conjunction with CT or MRI films on hand. But, the target area of the tumor determined in this way has some deviations from the actual area of the tumor. The target area of the tumor to be treated may exceed the tumor boundary or may be quite smaller than the area of the tumor according to the tumor boundary. For an operator with insufficient experiences, the deviations are larger. Such therapy system quite depends on the clinical experiences of the operator, the implementation of therapy becomes more complex and the uncertainty of therapeutic results becomes larger. Or, under relatively complex conditions, it is very difficult to implement the therapeutic procedures and the safety and effects of the therapy cannot be ensured. Thus it can be seen that it is very important to accurately localize the tumor boundary.

X-ray computed tomography scanner (CT)

CT (computed tomography) was a breakthrough in radiation diagnosis in the early 70's. CT uses X-ray to scan the body and gain the information. Then the information is processed by the computer and the reconstructed images can be gained. It can image the organs and diseases those are difficult to be imaged by conventional X-ray. These reconstructed images with definite anatomical relationship are like real, accordingly the checking scope for the body has been widened and the early detection rate and the accuracy rate of diagnosis of the diseases have been promoted greatly. This simple, convenient and safe scan without pain, trauma and danger has promoted the development of medical imaging diagnostics. CT was used to test head initially and the whole body CT scan appeared in 1974. During short ten years, CT has been used all over the world and has been developed from the first generation to the fifth generation. The whole body CT can take cross-section images of the head, chest, belly and pelvis. It also can take area scanning of small part of body, such as hypothyroid, spinal column, joints, soft tissue and five sense organs. CT is the most suitable to find out the occupied diseases, such as tumor, cyst, enlarged lymph node, hematoma, abscess and granuloma, and determine the size, modality, number and invasion range, also it can determine the staging of carcinomas of some organs. In some cases, CT also can identify pathological features of disease, such as solid, cystic, vascular, inflammatory, calcium-related, fat and etc. CT scan has three methods including plain scan, enhanced scan and contrast scan. The plain scan is a routine scan and a general check. The enhanced scan that can display some diseases more clear is to intravenously inject water soluble organic iodin before scanning. The contrat scan is to first apply contrast to an organ or a structure and then to scan it, for example, the contrast or air is injected into brain cistern and then the scanning begins so as to display the brain cistern and the small tumors in it.

The resolution of CT for tumor is higher than that of B-mode ultrasound. For a small tumor of 1 to 2 cm, its visualization rate for CT is 88% and for B-mode ultrasound is 48%. Particularly, CT is good at imaging brain hemorrhage, hydrocephalus, brain arterial malformations, brain cancer and etc. For gallbladder diseases, the diagnosis accuracy of B-mode ultrasound exceeds that of CT and normally the diagnosis accuracy of B-mode ultrasound is 95%. Also, for hepatocirrhosis, fatty liver, splenomegaly and intestinal diseases, the detection rate of B-mode ultrasound is high.

In the existing technology, already some ultrasound therapy systems deploy an immobilization means to link the coordinates system of CT scanning images with the coordinates system of therapy. CT images are used to make treatment procedures and then by the relationship between the coordinates system of CT scanning images and the coordinates system of therapy, the automatically controlled treatment is performed without image monitoring during treatment. The CT images here are the past images (not real-time images). Even though this method has improved the accuracy of localization to some extent, the deviation of target area to be treated may happen due to the physical movements, such as respiratory movement, heart beating, digestive organ movement and etc. The influences on the target area to be treated from this kind of movements can not be learned because of no monitoring, the treatment may still be deviated with great possibility. Meanwhile, it is very difficult to ensure the consistency of the body positions of CT scan and treatment without any monitoring. Generally, the human body is closed in a rigid immediately shaped phantom for a single use before CT scan, then the human body and the phantom are together scanned by CT. Because the human body is closed in the rigid phantom, the patient cannot take off the phantom for a rest so as to avoid damaging the phantom. The patient cannot be fixed in the closed phantom for a long time and the patient together with the phantom has to be transferred soon to therapy equipment for treatment after CT scan. This method requires that the therapeutic plan shall be made and the treatment shall be performed immediately or in a short time after CT scan.

Additionally, there are a lot of factors to influence the therapeutic effects due to characteristics of ultrasound therapy, for example, physical conditions of patient (for example, fatness of patient), position of tumor, tumor characteristics (for example, blood supply within tumor), tumor depth from skin, acoustic transmission way[0] blocking by ribs and etc. These factors directly influence the effects of ultrasound therapy. Many factors are difficult to be measured and have great influence on the therapy. For example, the blood supply within the acoustic transmission way and the target area to be treated have large effects on the temperature rise of the target area. But the blood supply is very difficult to be calculated. If there are no real-time evaluations on therapeutic effects and no timely adjustment of the therapeutic plan, the whole therapeutic effects of ultrasound therapy are limited.

Nuclear Magnetic Resonance Imaging (NMRI)

Nuclear Magnetic Resonance Imaging (NMRI) is an important application in biological and medical fields. It has a short name of MRI (Magnetic Resonance Imaging) and also refers to Nuclear Magnetic Resonance CT (CT here is short for computer tomography). The simple principle of MRI is: the patient lies inside an imaging magnet. Radio-frequency signals are then applied to the patient. The hydrogen nuclei in region of the subject are excited by radio-frequency signals and sends weak radio-frequency signals, which refer to nuclear magnetic resonance signals. During this process, the appropriate gradients are applied to the magnetic field so that the magnetic resonance signals can be acquired selectively. The information is processed to gain the tissue characteristics of each point and further the tissue can be imaged.

Magnetic Resonance Imaging (MRI) has great ability to identify different tissue and is easy to distinguish the normal tissue and tumor tissue and to determine the boundary of tumor tissue. MRI provides the volume data of a subject and a part of human body or full body can be imaged, therefore MRI is very suitable for locating the region of the subject to be treated by HIFU and planning HIFU surgical procedures. Meanwhile, with the development of MRI technology, the existing MRI equipment already can gain the images of the tissue in real-time, moreover the image is three-dimensional image with a certain volume. Therefore, MRI provides an excellent technical solution in monitoring the treatment procedures in real-time. Particularly, the temperature image provides a noninvasive temperature measuring method expected by the thermal therapy.

In this art, it has been discovered that the ultrasound therapy for internal tissue of patient is monitored and guided by MRI. In HIFU surgery, MRI may be used to scan the patient for locating the region of the subject to be treated before HIFU treatment and also to guide the ultrasound wave to the region of the subject and monitor the temperature changes of the tissue during HIFU treatment so as to ensure that only the region of the subject is heated without destroying the surrounding normal tissue. The advantages of MRI are well known by the technicians skilled in this art.

MRI can not only avoid the radiating effects of X-ray CT to human body but also image the diseased tissue. At present, MRI is a relatively ideal means to examine the bone, joints, spinal cord, viscera in pelvic cavity, uterus, mediastinum diseases, great vessels diseases of heart and to identify myocardial infarction.

But the application cost for MRI real-time monitoring is very high. Moreover, for the steadiness of magnetic field of MRI, the nonmagnetic designs for the therapy system have a high level; therefore, the therapy system is very difficult to be used combined with the MRI system.

To summarize, B-mode ultrasound, CT and MRI have their own advantages and also have their disadvantages. In addition, the image information limits due to their different imaging principles make the effects of single use of one kind of image not ideal. Therefore, the clinical therapy is urgent for technical solutions with a low cost, high performance and convenient implementation to solve the problem of localization in ultrasound therapy.

The inventor hopes to find a new image processing method to solve the problems stated as above. With rapid developments of computer, communication, sensors and material technologies, the image fusion appeared and has been developed since 1990's. Some breakthrough fruits in the field of image fusion have been achieved in recent years. Image fusion technology provides a method to process images.

Initially, the image fusion indicates that the images gained by the same or different imaging modalities are superposed so as to gain complementary information and increase information amount after these images are necessarily transformed geometrically and their spatial resolutions are unified and the positions are matched. While, the study scope of present image fusion includes image contraposition, display and analysis of fused images, effective correction and data reconstruction in emission data (SPECT, PET) using priori information gained from the corresponding anatomical images (MRI, CT).

Always, the information provided by different imaging modalities is complementary with each other. In order to use the different imaging modalities to provide more complete information, the effective information always needs to be integrated. The first process of the integration is to make the geometrical positions of multiple images correspond point by point in a spatial field. This process is referred as "registration". The second process of the integration is to combine the complementary information contained in the fused image and display the information. This process is referred as "fusion".

For different relatively complex clinical needs, a technical solution, which can be carried out with a low cost, easily match with the existing equipments with good effects and particularly is suitable for high-intensity focused ultrasound therapy system, is needed so as to improve the ultrasound therapeutic techniques and to enhance the safety and effectively shorten the treatment time. At present, there is no such technical solution disclosed in the field of ultrasound therapy.

## SUMMARY OF THE INVENTION

The object of the present invention is to provide a reliable method to localize the target area to be treated for HIFU therapy, which can enhance the safety and effectiveness of therapy and meanwhile can solve the technical difficulties in this therapy with a reasonable cost. Meanwhile, the present invention hopes to use this locating method to monitor the treatment as so to carry out the ultrasound therapy more safely. In order to realize the above-mentioned object, the present invention provides the technical solution as following.

One aspect of the present invention is to provide a focused ultrasound therapy system, comprising a central control means, which is used to control said system, includes a means to control acoustic energy range and move the therapeutic focal point and an interface device for inputting and outputting information to or from said therapy system and may input the operation commands using mouse and keyboard and observe the B-mode ultrasound images transferred from ultrasound guiding device by a display for determining the area to be treated; an acoustic energy applicator for applying the energy to a preset target area and generating acoustic energy and concentrating the acoustic energy within a small region, for example, a region of 0.3*0.3*1 cm$^3$, to form a therapeutic focal point; a mechanical driving and locating means of the acoustic energy applicator for moving the detection probe for imaging according to the instructions and moving said acoustic energy applicator to locate said therapeutic focal point; a real-time B-mode ultrasound image guiding device for scanning the target area, generating B-mode ultrasound images and transferring the generated B-mode ultrasound images to said central control means in real-time so as to make the operator locate the diseased part and apply acoustic energy for treatment according to said images. Said focused ultrasound therapy system further comprises an immobilization means for body position. By the help of this immobilization means for body position, the real-time B-mode ultrasound images can be aligned with one diagnosis image (or called as "registration"), and then on the basis of registration, B-mode ultrasound images are fused with the diagnosis images for guiding the therapy.

The diagnosis images mentioned in the one aspect of the present invention include, but not limited to CT images, MRI images, SPECT images, PET images or the registered and fused images by above-mentioned images.

The guided therapy adopted by the therapy system of the present invention is the therapy with treatment plan made manually. Or the 3-D treatment plan can be made using said diagnosis images, then said 3-D treatment plan is projected to the real-time B-mode ultrasound images and the automatic treatment plan is made. Also, the 3-D treatment plan can be made using the real-time acquired B-mode ultrasound images, then said 3-D treatment plan is projected to said diagnosis images to be modified and adjusted and the automatic treatment is carried out according to said adjusted plan.

Additionally, the system of the present invention may make 3-D treatment plan and perform the automatic treatment.

Further, the system of the present invention may adopt B-mode ultrasound for real-time evaluations on therapeutic effects.

Meanwhile, the present invention provides a locating means for image registration. This special made localization and immobilization means can be interfaced with diagnosis equipment and may be positioned on the table of diagnosis equipment, such as CT, MR and etc., to be examined and has no influence on imaging. This locating means is also designed to be interfaced with therapy equipment. With this locating means, the patient is fixed on the table of the therapy equipment and meanwhile, the consistency of body position for treatment and the body position for checking is ensured. For different diagnosis equipments, the different interfaces of the locating means are designed so as to ensure its best matching with the diagnosis equipments and its convenient installation.

By providing a focused ultrasound therapy system capable of more accurate localization, the present invention integrates the existed different medical diagnosis images and B-mode ultrasound images for monitoring so as to easily realize the interfaces with the existing ultrasound therapy system and particularly facilitate clinical localization of tumor, make treatment plan and monitor treatment in real-time. The operator can accurately find out the target area to be treated.

Beneficial effects of the present invention

Through the real-time B-mode ultrasound images and diagnosis images, such as the registration of CT or MRI images, the operator can accurately find out the target area to be treated. On the basis of images registration, the present invention makes the registered images, CT or MRI images fusion with real-time B-mode ultrasound images so as to better guide the operator to perform the therapy. On the basis of image registration and fusion and through making 3-D treatment plan, 3-D automatic treatment and real-time 3-D virtual treatment monitoring are performed. Comparing to the existed technical solutions in this field, the present invention has effectively solved the difficult problems in high-intensity focused ultrasound therapy and particularly for tumor treatment with a low cost. It provides a very practical technical solution, which can be easily applied to clinical treatment of tumors.

## BRIEF DESCRIPTION OF THE DRAWINGS

After the summary of the invention, the objects, advantages and implementation of the invention can be better understood by the technicians skilled in this art with reference to the following detailed description with accompanying drawings and embodiments.

Fig. 1 is a graph illustrating the ultrasound therapy system of the present invention.

Fig. 2A is an operational flow for registration and fusion of B-mode ultrasound image and CT for treatment of the present invention.

Fig. 2B is an operational flow for registration and fusion of B-mode ultrasound image and MRI for treatment of the present invention.

Fig. 2C is an operational flow for registration and fusion of B-mode ultrasound image and the fused image of CT and MRI for treatment of the present invention.

Fig. 3A is a diagram illustrating the installation position of the locating means of the present invention.

Fig. 3B is a diagram illustrating the locating means for registration of diagnosis images and B-mode ultrasound images. The locating means displayed here is a normal vacuum fix underlay.

Fig. 3C is a diagram illustrating the locating means for registration of diagnosis images and B-mode ultrasound images. The locating means displayed here is a vacuum fix underlay after vacuumized.

Fig. 4 is a diagram illustrating the image registration flow of the present invention, and

Fig. 5 is a diagram illustrating the treatment and installation of locating means on the treatment bed.

## DETAILED DESCRIPTION OF PREFERRED EMBODIMENTS

### Embodiment 1

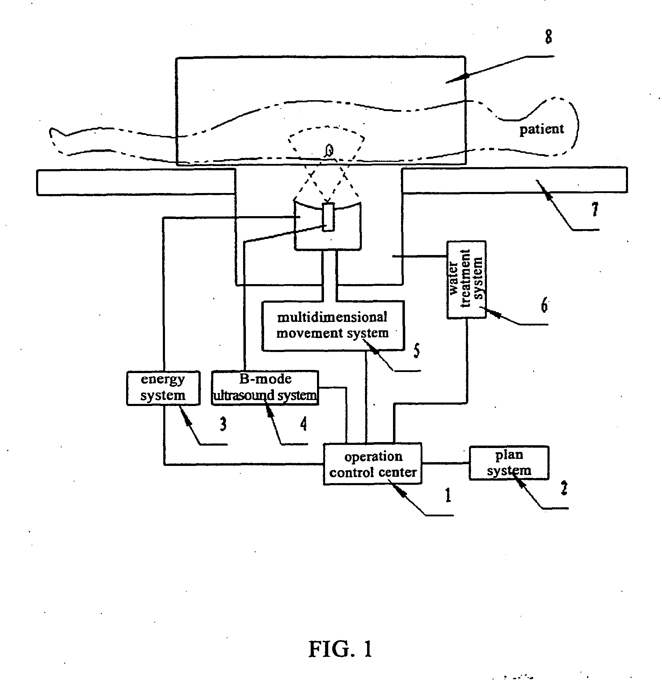

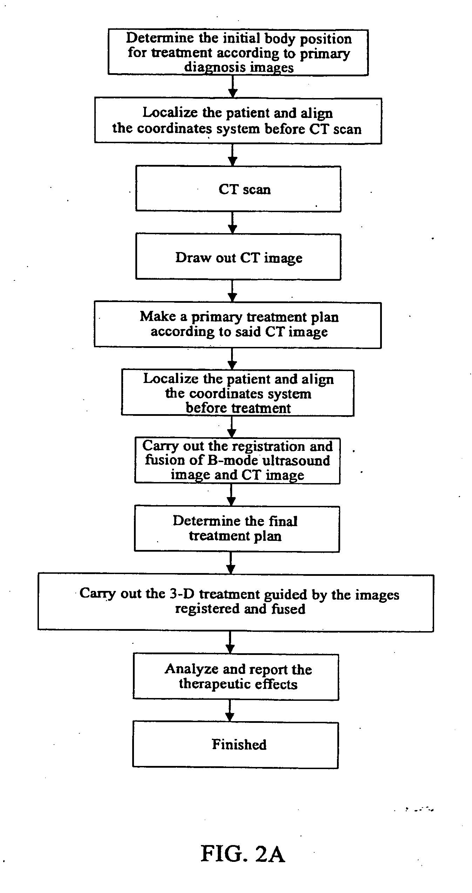

As shown in Fig. 1 and Fig. 2A, the embodiment of the present invention includes operation control system **1**, 3-D treatment plan system **2**, energy controller **3**, B-mode ultrasound system **4**, multidimensional movement system **5**, water treatment system **6**, treatment bed **7** and locating means **8** and etc..

In embodiment 1 of the present invention, the diagnosis image is CT image with a relatively high resolution. There are many kinds of existing CT scanners in the market and the products of GE, Philips, Siemens, Toshiba and etc., for example, LightSpeed 16 from GE, can be selected. For the relative information, refer to **http://www.gehealthcare.com/cnzh/rad/ct/products/light_series/index.h tml.**

The CT image and B-mode ultrasound image of the patient (the B-mode scanner may adopt ESAOTE DU4, see *http:llwww.esaote.com.cnlproduct.asp)* are registered and fused so as to guide the operator to perform the treatment. Or, according to the registered and fused image, 3-D treatment plan is made and the automatic treatment under the monitoring by operator is carried out.

The operation flow of embodiment 1 of the present invention is illustrated in Fig. 2A.

At first, the initial body position for treatment according to primary diagnosis images is determined and a virtual treatment on therapy equipment under monitoring by B-mode scanner is performed and the optimal body position for treatment is determined. This process is referred as pre-positioning. After pre-positioning, the tumor can be examined by the diagnosis imaging with high resolution, for example, CT or MRI, or the functional imaging, for example, PET. Before any examination, the patient needs to be fixed according to preset body position and the coordinates system shall be aligned. After examination, the image can be drawn out and a primary treatment plan can be made according to that image. Before implementation of treatment, the patient is localized on the therapy equipment according to preset body position. After localization, the image registration and fusion are carried out and the final treatment plan is determined. Then the treatment guided by the images registered and fused in real-time and under the monitoring of 3-D virtual actual system can be carried out. After the treatment is finished, the therapeutic effects are analyzed and evaluated and a report is issued.

### Embodiment 2

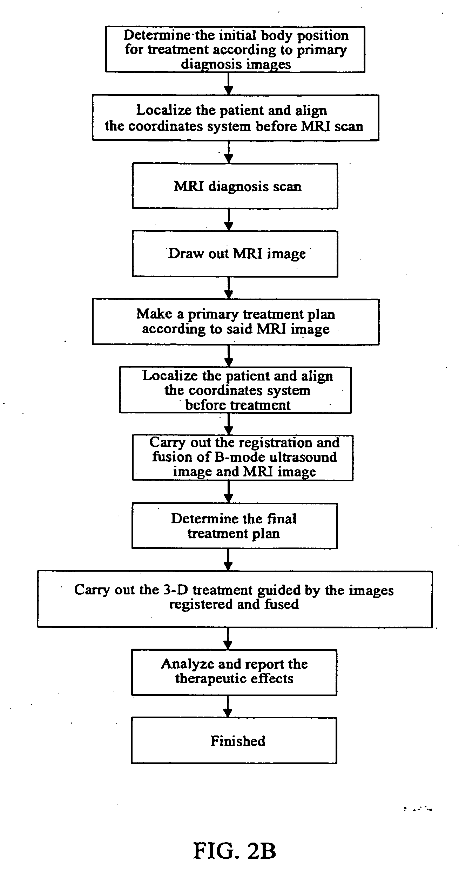

For embodiment 2 of the present invention, refer to Fig. land Fig. 2B. The MRI image for diagnosis with a relatively high resolution and B-mode ultrasound image of the therapy system are registered and fused so as to guide the operator to perform the treatment. Or, according to the registered and fused image, 3-D treatment plan is made and then the automatic treatment under the monitoring by operator is carried out.

This MRI image can be gained from the equipments sold in the market, for example, Signa MR/i 1.0/1.5T from GE (referring to http://www.gehealthcare.com/cnzh/rad/mri/products/mri/mri.html).

### Embodiment 3

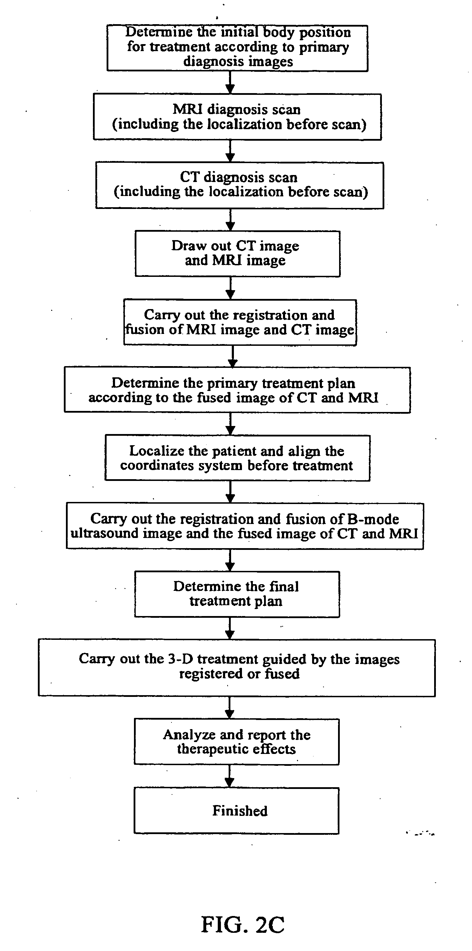

For embodiment 3 of the present invention, refer to Fig. 1 and Fig. 2C. The fused image of CT and MRI with a relatively high resolution and B-mode ultrasound image of the therapy system are registered and fused so as to guide the operator to perform the treatment. Or, according to the registered and fused image, 3-D treatment plan is made and then the automatic treatment under the monitoring by operator is carried out.

Before fusion of CT and MRI images, CT scan and MRI scan on patient are respectively performed. When scanning, the locating means 8 shall be used for localization and immobilization.

Other procedures are the same as those in embodiment 1.

The common working procedures of the embodiments of the invention

The other processing procedures of the invention are described in detail thereinafter.

Initial positioning and pre-positioning

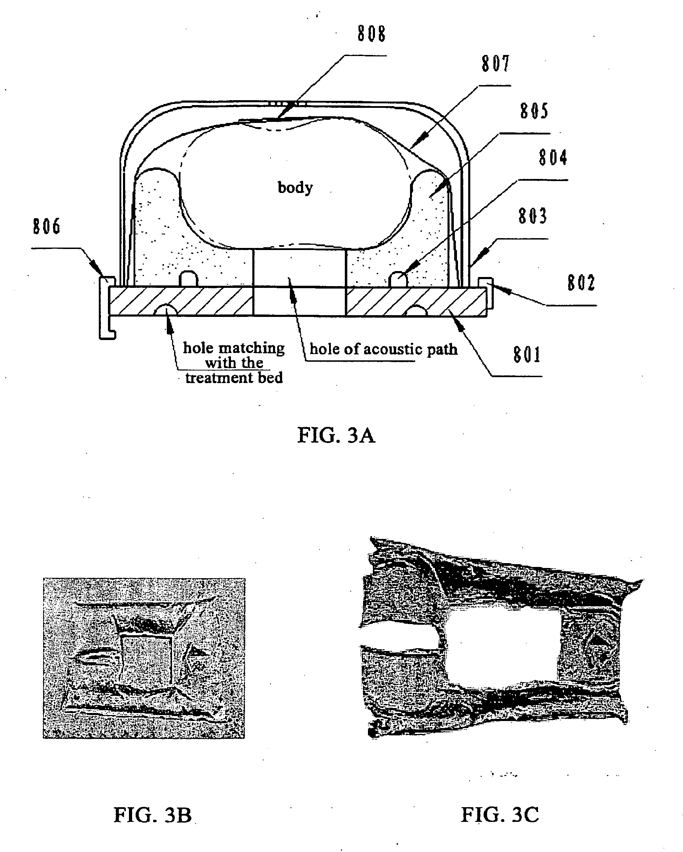

The operations of initial positioning and pre-positioning are very simple and may have been used in many treatment modes before. The operator gains the initial diagnosis images, which may be existed in a form of film or CD. From these images the operator can determine the size and position of the tumor and according to operator's own experience can initially determine the body position with which the good therapeutic effects can be achieved and the acoustic energy may be fully focused in tumor region without damaging the surrounding normal tissue and dangerous organ. Then, the operator will make pre-position on the therapy equipment of the invention and use B-mode ultrasound on the equipment to monitor so as to determine appropriate body position for treatment. And the vacuum mat 802 in Fig. 3 is used for figuration and immobilization. And the locating and fitting mark shall be made normally on the body skin, which is difficult to move, for example, skin of ehest. Or, the marks are made according to the end position of bone.

Realization of image registration

The present invention uses the locating means 8 together with simple calculations to realize the image registration. The registration is only the horizontal movement and zoom scale. The arithmetic is described as below. Note: All kinds of coordinates Systems described as below are 3D Cartesian Coordinates Systems. The direction of each axis is the same and only the positions of original points are different.

The offset of the coordinates System of imaging relative to the coordinates system of the locating means is Offset1(x,y, z); wherein, the determination of Offseti is explained thereinafter (formula 7).

The offset of the coordinates System of the locating means relative to the coordinates system of therapy equipment is Offset2(x, y, z), wherein, the determination of Offset2 is explained thereinafter.

The space (mm) occupied by one pixel dot of the diagnosis image, i.e. the scale is PixelSpacingc(x, y, z). The x component and y component of this scale can be measured by the scale on the diagnosis image. Z component is equal to the distance between slices to be scanned or can be read from the Standard medical image file of DICOM (Digital Imaging and Communications in Medicine). At present, all the mainstream diagnosis imaging equipments provide the medical images with this format.

After the unit of pixel is transformed into "mm" unit, any point of Pc (x ,y, z) in diagnosis image becomes a point of P (x, y, z). The coordinates of that point in the locating means is P1, so

$$P.x = Pc.x * PixelSpacingc.x;$$

$$P.y = Pc.y * PixelSpacingc.y;$$

$$P.z = Pc.z * PixelSpacingc.z; \quad (Formula\ 1)$$

If P is known, find Pc as below:

$$Pc.x = P.x / PixelSpacingc.x;$$

$$Pc.y = P.y / PixelSpacingc\ .y;$$

$$Pc.z = P.z \,/\, PixelSpacingc.z; \quad (\text{Formula 2})$$

From P find P1 as below:

$$P1.x = P.x + Offset1.x;$$

$$P1.y = P1.y + Offset1.x;$$

$$P1.z = P1.z + Offset1.y; \quad (\text{Formula 3})$$

The coordinates of P point in the therapy coordinates system is P2, so

$$P2.x = P1.x + Offset2.x = P.x + Offset1.x + Offset2.x$$

$$P2.y = P1.y + Offset2.y = P.y + Offset1.y + Offset2.y$$

$$P2.z = P1.z + Offset2.z = P.z + Offset1.z + Offset2.z \quad (\text{Formula 4})$$

If any point of P2 in the therapy coordinates system is known, find the point of P in the coordinates system of diagnosis image as below:

$$P.x = P2.x - (Offset1.x + Offset2.x)$$

$$P.y = P2.y - (Offset1.y + Offset2.y)$$

$$P.z = P2.z - (Offset1.z + Offset2.z) \quad (\text{Formula 5})$$

The point of Pc with a unit of pixel is calculated using (formula 2).

According to the point of Pb in the B-mode ultrasound image the coordinates of P2 of that point in the therapy coordinates system can be found,

The space (mm) occupied by one pixel dot in the B-mode ultrasound image, i.e. the scale is PixelSpacingb(x, y, z). The x component and y component of this scale can be directly measured by the scale on the B-mode ultrasound image. Z component is equal to the distance between slices to be scanned.

The offset of the coordinates system of B-mode ultrasound image relative to the therapy coordinates system is Offsetb(x, y, z). Offsetb is determined by the installation of B-mode probe of the therapy equipment. Because it is decided by the therapy equipment, there is no need of any calculations.

$$P2.x = Pb.x * PixelSpacingb.x \quad + Offsetb.x$$

$$P2.y = Pb.y * PixelSpacingb.y \quad + Offsetb.y$$

$$P2.y = Pb.y * PixelSpacingb.y \quad + Offsetb.y \quad （Formula\ 6）$$

After P2 is found and P can be calculated using (formula 5) and then its Pc in imaging coordinates system is calculated using (formula 2).

When registration, generally, we first acquire a B-mode ultrasound image in real-time. If the registration is performed in an area, the area is supposed to be set as a rectangle region (Z-coordinate of all points in the same image are the same), which is determined by x, y components of the points Pb1 and Pb2.

The coordinates Pc1 and Pc2 of Pb1 and Pb2 in the diagnosis image are calculated respectively using (formula 6), (formula 5) and (formula 2), then the image within the rectangle region determined by x, y components of Pc1 and Pc2 is acquired. Because the scale PixelSpacingb of B-mode ultrasound image is possibly different from the scale PixelSpacingc of diagnosis image, the acquired diagnosis image shall be zoomed with a zooming coefficient of PixelSpacingc/ PixelSpacingb (formula 7). After the processing as above, there is a corresponding registered diagnosis image in a certain area of the B-mode ultrasound image and accordingly the image registration is realized.

The human body is considered as a rigid subject when the locating means **8** is used. The B-mode scanner, CT or MR are used to acquire the images of this rigid subject. Because the coordinates, size and scale of B-mode ultrasound images are different from those of CT or MR images, the transformations of coordinates, size and scale are needed. The offset is got by the locating means.

As shown in Fig. 3A, the locating means **8** for image registration mainly comprises positioning plate **801,** treatment locating mark **802,** locating carriage **803,** locating pillar **804,** vacuum fix underlay **805,** treatment locking means **806** and binding strip **807** and seta buckle on the binding strip (nylon agraffe, hook and loop) **808.**

The locating means 8 is used to fix the patient like a rigid subject. In different time, the spatial position of patient body can be changed, but the patient body isn't distorted.

Image registration

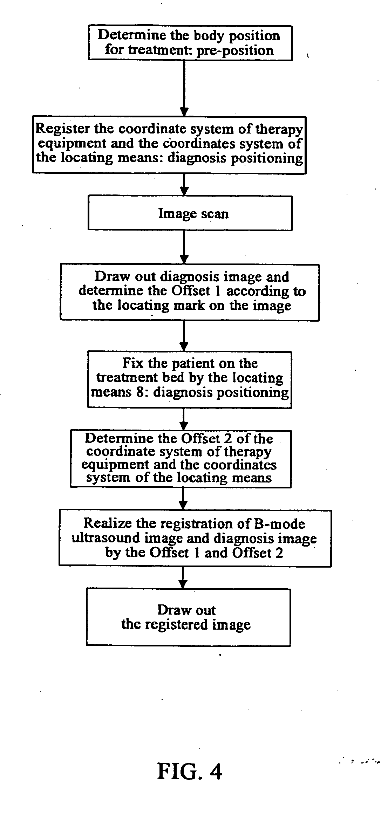

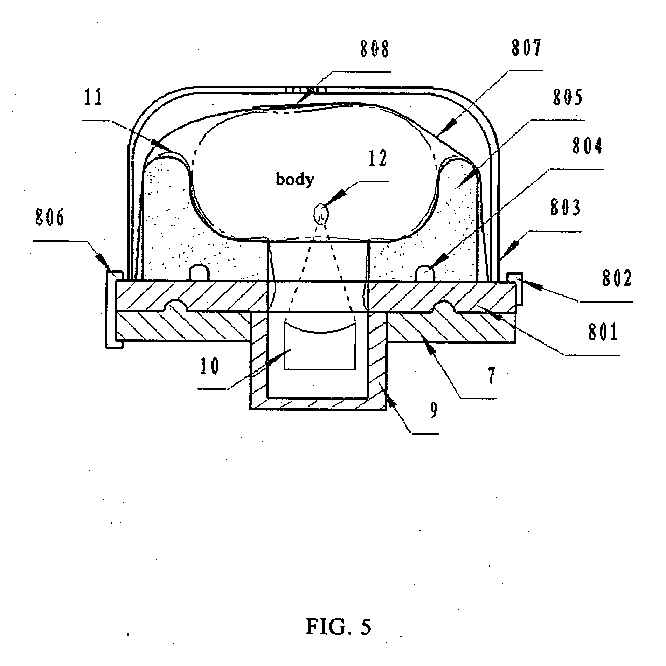

The operation flow for image registration is shown in Fig. 4. The principle, installation and operation of locating means 8 are illustrated in Fig. 5.

Locating means **8** includes the positioning plate **801** for installation interface between the therapy equipment and the diagnosis equipment; the locating pillar **804** installed on the upper surface of the positioning plate **801** for determining the position of the vacuum fix underlay relative to the positioning plate **801**; the vacuum fix underlay **805** used between the human body and the positioning plate and placed on the positioning plate 801. The locating pillar 804 is used to determine the horizontal position of the mat. The human body lies on the vacuum fix underlay 805 and the body surface contacts to the vacuum fix underlay closely. After the vacuum fix underlay 805 is vacuumized, it is shaped with rigidity and can fix and locate the patient. One end of the binding strip **807** is fixed on the positioning plate **801.** The seta buckle at the other end of the binding strip **807** fast adheres together with the seta buckle of another binding strip. Treatment locating mark 802 is used to determine the offset of the locating means along the axial direction of human body (Z-coordinate) relative to the treatment bed. It is located at the zero-position of locating coordinates system. Treatment locking means 806 is used to fix the positioning plate 801 on the treatment bed 7 in order to avoid the movement of positioning plate relative to treatment bed 7 during treatment.

For the vacuum fix underlay, refer to http://www.topslane.com.cn/pro02-VFUc.html. The present invention adopts the vacuum fix underlay produced by Topslane International, Inc. It assists the operator for positioning and repositioning the patient with tumor. The vacuum cushion is filled up with a special granular material. The vacuum cushion is soft under ordinary pressure so as to make a patient be mold easily. When the air in the vacuum cushion is taken out, the vacuum cushion hardens gradually adaptive to the body contour of patient and after a few minutes it becomes a rigid mold fully adaptive to the patient anatomical contours. In this way, the accuracy of treatment localization and positioning can be ensured and the time for localization and positioning can be decreased so as to improve the working efficiency. After being taken out air, the vacuum fix underlay is capable of keeping shaped for a long time and normally around 30 days. The different forms of vacuum fix underlay under different conditions are illustrated in Fig. 3B and Fig. 3C.

The positioning plate **801** is designed according to the treatment bed of the ultrasound therapy equipment and the diagnosis equipment in order to ensure the smooth installation without any interference with the movements of the therapy equipment and the diagnosis equipment.

Installation and positioning of locating means 8 on the therapy equipment,

The pre-positioning is illustrated in Fig. 5. There is a hole designed at the middle of the positioning plate **801** and also there is a corresponding hole at the middle of the vacuum fix underlay so that the acoustic energy emitted from the treatment applicator 10 can be transmitted into the human body without any obstruction and a therapeutic focal point 12 is formed at the diseased region of a patient. There is a matching groove on the lower surface of the positioning plate **801** so as to ensure the matching with the raised portion of the treatment bed for localization. After the positioning plate **801** matches well with the treatment bed, the coordinates system of the locating means is related to the coordinates system of therapy equipment. Commonly the origins of x and y and directions of both the coordinates system of locating means and therapy equipment are superpositioned and only Z-coordinate in vertical direction has an offset. This offset SP can be read from the Z-coordinate of the coordinates system of the therapy equipment through the mark 802.

The offsets in installing in x, y, z-direction are the offsets of the coordinates system of the locating means relative to the coordinates system of the therapy equipment, i.e. Offset2(x, y, z); Offset2.x=0; Offset2.y=0; Offset2.z = SP.

After the positioning plate **801** is installed on the treatment bed, the vacuum fix underlay 805 under normal state is placed on the positioning plate **801.** Because the vacuum fix underlay 805 at this time is soft, the locating pillars will be enwrapped by the vacuum fix underlay and the locating hole is formed along the locating pillars after the air in the vacuum fix underlay is taken out. After the vacuum fix underlay 805 is mounted, then the patient is placed on the vacuum fix underlay and the body surface around the area to be treated shall be aligned with the holes of the vacuum fix underlay 805 and the positioning plate **801.** Then, the binding strip **807** is used to evenly bind the soft vacuum fix underlay together with the patient. And then the air in the vacuum fix underlay is taken out by using a vacuum pump in order to finalize its shape. After the shape is finalized, the operator uses B-mode ultrasound image to observe the diseased part and make a judgment if this body position is reasonable. If it is unreasonable, some air needs to be pumped into the vacuum fix underlay until it becomes soft. Then the body position of the patient shall be readjusted and the air in the vacuum fix underlay is taken out again until the appropriate body position is found. After the appropriate body position is determined, a matching mark shall be made on the human body and the vacuum fix underlay by using a mark pen so as to facilitate the checking and correction when repositioning. Then, the installation and position of patient on the therapy equipment has been finished. This locating process is referred as treatment pre-positioning or initial positioning.

After the initial positioning is completed, the binding strip is released. Even through the vacuum fix underlay is shaped, it still has some elasticity and it can recover after its appropriate distortion. Additionally, the vacuum fix underlay isn't fully closed. Therefore, the patient can be taken out easily from the vacuum fix underlay without demolishment to the shape of vacuum fix underlay. Then the vacuum fix underlay is taken away from the positioning plate for saving and the bleed hole shall be maintained in a closed status. Finally the positioning plate 801 is taken away from the treatment bed.

Then, the locating means 8 is installed and the patient is positioned on the diagnosis equipment.

Installation and positioning of locating means on the diagnosis equipment (diagnosis positioning)

The patient and the locating means are brought together to the examination room. Firstly the positioning plate 801 is installed on the diagnosis equipment, for example, MR or CT. Its installation is simple and the positioning plate 801 is directly placed on the diagnosis equipment and the positioning plate shall be kept parallel to the diagnosis equipment without any incline. There is no need of locking between the positioning plate **801** and the examination bed and the friction between the positioning plate and the examination bed can enough ensure no movement of the positioning plate relative to the examination bed during the checking. Then, the shaped vacuum fix underlay **805** is placed on the positioning plate **801** and the locating hole of the vacuum fix underlay shall match with the locating pillar **804** on the positioning plate. Then, the patient is put into the vacuum fix underlay and the matching marks on the human body and the vacuum fix underlay shall be checked carefully. Finally the binding strip is used to bind the vacuum fix underlay with the human body in order to avoid any shaking.

After the positioning of diagnosis and examination has been finished, the scanning can be performed by the operator of diagnosis equipment.

After the examination has been completed, the binding strip 807 is released and the patient is taken out from the vacuum fix underlay. Meanwhile, the operator of the diagnosis equipment records the image data to CD for later image analysis and initial treatment plan and for making preparations for B-mode ultrasound image registration.

The Offset2 of the coordinates system of the locating means relative to the coordinates system of the therapy equipment has been determined and the Offset1(x, y, z) of the coordinates system of diagnosis image relative to the coordinates system of locating means is determined as below.

The Offset1 is determined by image mark identification.

The locating carriage 803 is arranged on the locating means. The locating carriage **803** has at least one marker. Each marker has a fixed coordinate Ps in the coordinates system of the locating means. These markers have a gray scale different from the tissue of human body in the diagnosis image. They are displayed as a bright point or a dark point on the image. For example, the marker in the CT imaging may be a metal, for example, iron and it is a bright point on the CT image. Through identification, these marks can be circled on the images displayed on the computer by the software automatically or by the operator manually using a mouse. The software can calculate the coordinates Pcs of these markers in the image according to the position of a mouse, so

$$\text{Offset1.x} = \text{Ps.x} - \text{Pcs.x} * \text{Pixelspacingc.x}$$

$$\text{Offset1.y} = \text{Ps.y} - \text{Pcs.y} * \text{Pixelspacingc.y}$$

$$\text{Offset1.z} = \text{Ps.z} - \text{Pcs.z} * \text{Pixelspacingc.z} \quad （\text{Formula 7}）$$

Positioning when treatment (Treatment positioning)

Referring to Fig. 5, firstly the positioning plate 801 is fixed on the treatment bed 7. The operations here are the same as that of pre-positioning. The shaped vacuum fix underlay 805 is placed on the positioning plate **801** and the locating hole of the vacuum fix underlay 805 shall match with the locating pillar **804** on the positioning plate 801. Then, the water seal cloth 11 for fixing the water bag 9 of the therapy equipment covers the vacuum fix underlay 805 and the lower open of the water seal cloth 11 is fixed on the brims of the water bag in order to seal the water. The water seal cloth 11 is thin and only less than 0.1mm thick, therefore, adding one layer of the water seal cloth 11 will not influence the treatment relative to the examination positioning. Then, the patient is fixed on the vacuum fix underlay 805 and the water seal cloth 11 and the matching marks on the patient and the vacuum fix underlay shall be checked carefully. Finally the binding strip 807 is used to bind the patient, the vacuum fix underlay 803 and the positioning plate 801 together. When the treatment positioning is performed, the installation of locating means sometimes is different from that in pre-positioning, therefore, the Offset2 is subject to the results of this positioning operation.

The locating means ensures that the spatial position change is only the horizontal movement without any rotation. This makes the registration of diagnosis image and B-mode ultrasound image simpler. It doesn't need complex image arithmetic. It is a registration depending on mechanical assurance with a high reliability

Normally, it is very complicated to realize image registration. The normal registration method includes the following steps: feature extraction, feature matching, transformation option and parameters determination and implementing whole transformation. Each step needs a lot of manipulations and operations. Taking feature extraction as an example, when the same marker, for example, sternum is extracted respectively from two images to be registered, the approximate position of sternum on the image or the features of sternum, for example, gray scale feature or texture feature shall be given clearly by the operator, then the computer is used to perform operations and extraction according to given features. Even though there are some kinds of software for automatic registration at present, the quantity of operations is large and the accuracy isn't high. Particularly for B-mode ultrasound image, the features of each tissue are not very obvious and the automatic registration is more difficult. At present, there is no software for automatic registration of B-mode ultrasound image and other diagnosis images. But the system of the present invention uses an immobilization means for body position to make only horizontal movement of position and simple zooming existed between two kinds of images to be registered. The operations of registration are very simple with the arithmetic as above described.

Certainly, due to status changes of human body itself or the physical movement of viscera and if the physical status of the patient when imaging is different from that when treatment, a little deviation of above mentioned images, which fully rely on mechanical registration, may be caused. Therefore, during the scanning of B-mode scanner on the therapy equipment, the breath of patient requires to be restrained and normally the scanning is carrying out when the inspiration finishes or exhalation finishes. Because the patient cannot have breath during scanning, the scanning has to be completed in a relatively short time. This kind of deviation may be detected through comparing the organ with an obvious mark in both images and then can be corrected by software functions for accurate registration.

Image fusion

After the images are registered, we can use the computer technology to fuse B-mode ultrasound image and other diagnosis image and to display two images in a same position. In this way, on the fused B-mode ultrasound image we can clearly find out the tissue or tumor, which can be seen only on other diagnosis image before fusion. On the basis of image registration, two images are fused only according to fusion arithmetic and then are displayed. The operator can directly use the fused image as a basis to accurately and fast determine tumor border and the target area to be treated and to perform a reasonable and effective treatment.

Image fusion display is one kind of the computer image processing. There are many image fusion methods and we adopt the two-dimensional fusion method based on the slices. There are some relative simple effective methods as below:

1. Direct fusion: Two images are superpositioned transparently and the scales of display intensity of two images are controlled;
2. Color components superposition method: Each color represented by computer has three components (R, G, B). One or two components of the first image are replaced by gray levels of the second image. In this way, the superpositioned image is a colorful image;
3. The image generated by the second method is transformed into a gray image; and
4. Spacing choice: The pixels spacing one or several pixels in the first image are replaced with the pixels with corresponding coordinates in the second image and a new image is gained.

One or several methods can be selected for image fusion according to actual applications.

Treatment method after image registration:

If the tumor is small and there is no critical tissue around it, 3-D treatment plan may not be used. The operator can determine the target area to be treated according to the registered or fused images and apply appropriate dose for treatment according to experiences.

The registered image is used to determine the target area to be treated. The target area to be treated may be determined by B-mode ultrasound image and meanwhile this area may be displayed in the registered CT image. The operator can first determine the target area to be treated in B-mode ultrasound image and check the target area in the registered CT image. If there is any difference found, the treatment can be carried out after appropriate adjustment. Also, the target area to be treated may be determined by the registered CT image and meanwhile this area may be displayed in the real-time B-mode ultrasound image. The operator can check the target area to be treated in the B-mode ultrasound image. If there is any difference found, the treatment can be carried out after appropriate adjustment.

3-D treatment plan on the basis of image registration or fusion

When the patient has finished the examinations by diagnosis equipments, such as CT or MRI and the checking images have been gained, then we can use these checking images to perform image segmentation and three-dimensional reconstruction. On the basis of three-dimensional reconstruction, the tumor border, the tissue in the acoustic pathway and the critical organs are analyzed and the treatment path and therapeutic dose are determined in conjunction of ultrasound therapy experts database. And then a comprehensive treatment plan is made.

After the image registration, the treatment plan made as above is transformed into the coordinates system of the therapy equipment by use of registration relationship. And a simulation treatment is carried out in 3-D simulated therapy equipment in order to check the reasonability of treatment plan and to evaluate or modify the treatment plan. After the simulation treatment is passed, the treatment plan is sent to the treatment control system. The operator can use real-time B-mode imaging system to monitor the treatment and to evaluate the therapeutic effects and to carry out the automatic or semi-automatic treatment by the help of 3-D virtual treatment monitoring.

The technicians skilled in the art may easily make numerous changes and improvements of the embodiments described as above or apply to other fields. This invention includes all kinds of embodiments and applications. Even through this invention is described according to the preferred embodiments, therefore the scope of the invention is not to be restricted, except by the following claims of this invention.

**Claims**

**1.** A focused ultrasound therapy system, comprising:

a. a central control means (1) for controlling said system and including a means (3) to control acoustic energy range and move a therapeutic focal point and an interface device for inputting and outputting information to or from said therapy system;

b. an acoustic energy applicator for applying the energy to a preset target area and forming a therapeutic focal point;

c. a mechanical driving (5) and locating means (8) of said acoustic energy applicator for moving a detection probe for imaging according to instructions and moving said acoustic energy applicator to locate said therapeutic focal point;

d. a real-time B-mode ultrasound image guiding device (4) for scanning the target area, generating real-time B-mode ultrasound images and transferring the generated B-mode ultrasound images to said central control means in real-time so as to make the operator locate the diseased part and apply acoustic energy to it according to said images;

**characterized in that** said focused ultrasound therapy system further comprises

an immobilization means for body position, which includes a positioning plate, a treatment locating mark, a locating carriage, a vacuum fix underlay as well as treatment locking means and a binding strip,

means for generating diagnosis images,

means for registering said real-time B-mode ultrasound images with said diagnosis images such that said real-time B-mode ultrasound images are aligned with said diagnosis images and

means for fusing said B-mode ultrasound images with said diagnosis images on the basis of registration for guiding said therapy.

2. The system as claimed in claim 1 wherein said diagnosis images are selected from CT image, MRI image, SPECT image, PET image or registered and fused images by above-mentioned images.

3. The system as claimed in claim 2 wherein said guided therapy is the therapy with treatment plan made manually.

4. The system as claimed in claim 2 wherein a 3-D treatment plan can be made using the said diagnosis images and said 3-D treatment plan is projected to the real-time B-mode ultrasound images for making an automatic treatment plan.

5. The system as claimed in claim 2 wherein a 3-D treatment plan can be made using the real-time acquired B-mode ultrasound images and said 3-D treatment plan is projected to said diagnosis images for modifying and adjusting and the automatic treatment is carried out according to said adjusted plan.

6. The system as claimed in claim 2 wherein said guided therapy is an automatic treatment with a 3-D treatment plan.

7. The system as claimed in any of Claims 2-6 wherein further uses B-mode ultrasound monitoring to evaluate the therapeutic effects in real-time.

**Patentansprüche**

1. Therapiesystem mit fokussiertem Ultraschall, umfassend:

a. eine zentrale Steuereinrichtung (1) zum Steuern des Systems, und mit einer Einrichtung (3) zum Steuern des Bereichs akustischer Energie und zum Bewegen eines therapeutischen Brennpunktes und einer Schnittstelleneinrichtung zum Eingeben und Ausgeben von Informationen in das oder aus dem Therapiesystem;

b. einen Applikator für akustische Energie zum Einbringen der Energie in einen vorgegebenen Zielbereich und zum Ausbilden eines therapeutischen Brennpunktes;

c. eine Einrichtung zum mechanischen Antrieb (5) und zum Positionieren (8) des Applikators für akustische Energie zum Bewegen einer Detektionssonde zur Bildgebung entsprechend Anweisungen und zum Bewegen des Applikators für akustische Energie zur Positionierung des therapeutischen Brennpunktes;

d. eine in Echtzeit arbeitende Ultraschall-Bildführungseinrichtung (4) für B-Modus zum Scannen des Zielbereichs, welche in Echtzeit Ultraschallbilder im B-Modus erzeugt und die erzeugten B-Modus-Ultraschallbilder in Echtzeit an die zentrale Steuereinrichtung überträgt, so dass der Bediener den krankhaften Teil lokalisieren kann und entsprechend den Bildern akustische Energie in diesen einbringen kann;

**dadurch gekennzeichnet, dass** das Therapiesystem mit fokussiertem Ultraschall ferner umfasst:

eine Immobilisierungseinrichtung zur Körperpositionierung, die eine Positionierungsplatte, eine Behandlungspositionsmarkierung, einen Positionierungsschlitten, eine Vakuumfixierungsunterlage sowie Behandlungsfesthaltemittel und einen Bindegurt umfasst,
Mittel zum Erzeugen von Diagnosebildern;
Mittel zum passgenauen Ausrichten der Echtzeit-Ultraschallbilder im B-Modus mit den Diagnosebildern in solcher Weise, dass die B-Modus-Echtzeit-Ultraschallbilder mit den Diagnosebildern ausgerichtet sind, und
Mittel zum Fusionieren der B-Modus-Ultraschallbilder mit den Diagnosebildern auf Grundlage der passgenauen Ausrichtung zum Führen der Therapie.

2. System nach Anspruch 1, wobei die Diagnosebilder ausgewählt sind aus CT-Bildern, MRI-Bildern, SPECT-Bildern, PET-Bildern oder zueinander ausgerichteten und fusionierten Bildern aus den oben genannten Bildern.

3. System nach Anspruch 2, wobei die geführte Therapie eine Therapie mit einem manuell erstellten Behandlungsplan ist.

4. System nach Anspruch 2, wobei mit Hilfe der Diagnosebilder ein 3D-Behandlungsplan erstellt werden kann und der 3D-Behandlungsplan auf die Echtzeit-Ultraschallbilder im B-Modus projiziert wird, um einen automatischen Behandlungsplan zu erstellen.

5. System nach Anspruch 2, wobei mit Hilfe der in Echtzeit erfassten Ultraschallbilder im B-Modus ein 3D-Behandlungsplan erstellt werden kann und der 3D-Behandlungsplan auf die Diagnosebilder projiziert wird, zur Modifizierung und Anpassung, und wobei die automatische Behandlung entsprechend dem angepassten Plan durchgeführt wird.

6. System nach Anspruch 2, wobei die geführte Therapie eine automatische Behandlung mit 3D-Behandlungsplan ist.

7. System nach einem der Ansprüche 2 bis 6, bei welchem ferner eine B-Modus-Ultraschall-Überwachung genutzt wird, um die therapeutischen Wirkungen in Echtzeit zu bewerten.


**Revendications**

1. Système de thérapie par ultrasons focalisés, comprenant :

a) un moyen de commande centrale (1), destiné à commander ledit système et comprenant un moyen (3) de commande de la plage d'énergie acoustique et de déplacement du point focal thérapeutique et un dispositif d'interface destiné à entrer et sortir des informations à destination et en provenance dudit système de thérapie ;
b) un applicateur d'énergie acoustique, destiné à appliquer l'énergie à une zone cible prédéterminée et formant un point focal thérapeutique ;
c) un moyen de déplacement mécanique (5) et de positionnement (8) dudit applicateur d'énergie acoustique, destiné à déplacer une sonde de détection destinée à produire des images selon des instructions et à déplacer ledit applicateur d'énergie acoustique afin de positionner ledit point focal thérapeutique ;
d) un dispositif de guidage (4) par imagerie ultrasonore en mode B en temps réel, destiné à balayer la zone cible, à produire des images ultrasonores en mode B et en temps réel et à transférer les images ultrasonores en mode B ainsi produites vers ledit moyen de commande centrale en temps réel afin de permettre à l'opérateur de localiser la partie malade et d'y appliquer l'énergie acoustique en fonction desdites images ;

**caractérisé en ce que** ledit système de thérapie par ultrasons focalisés comprend en outre :

un moyen d'immobilisation destiné à positionner le corps, comprenant une plaque de positionnement, un repère de positionnement du traitement, un chariot de positionnement, un coussin d'immobilisation à dépression ainsi qu'un moyen de verrouillage du traitement et une courroie de fixation ;
un moyen destiné à produire des images diagnostiques ;
un moyen destiné à caler lesdites images ultrasonores en mode B et en temps réel avec lesdites images diagnostiques, afin que lesdites images ultrasonores en mode B et en temps réel soient alignées sur lesdites images diagnostiques ; et
un moyen destiné à fusionner lesdites images ultrasonores en mode B et lesdites images diagnostiques sur la base de l'alignement, afin de guider ladite thérapie.

2. Système selon la revendication 1, dans lequel lesdites images diagnostiques sont sélectionnées parmi des images tomodensitométriques, des images IRM, des images SPECT, des images PET ou des images calées et fusionnées à partir des images mentionnées ci-dessus.

3. Système selon la revendication 2, dans lequel ladite thérapie guidée est une thérapie avec plan de traitement défini manuellement.

4. Système selon la revendication 2, dans lequel un plan de traitement tridimensionnel peut être défini en utilisant lesdites images diagnostiques et ledit plan de traitement tridimensionnel est projeté sur les images ultrasonores en mode B et en temps réel afin de réaliser un plan de traitement automatique.

5. Système selon la revendication 2, dans lequel un plan de traitement tridimensionnel peut être défini en utilisant les images ultrasonores en mode B et en temps réel, ledit plan de traitement tridimensionnel est projeté sur lesdites images diagnostiques dans un but de modification et d'ajustement et le traitement automatique est réalisé en fonction dudit plan ajusté.

6. Système selon la revendication 2, dans lequel ladite thérapie guidée est un traitement automatique avec un plan de traitement tridimensionnel.

7. Système selon l'une quelconque des revendications 2 à 6, utilisant en outre une surveillance ultrasonore en mode B pour évaluer les effets thérapeutiques en temps réel.

FIG. 1

```
┌─────────────────────────────────┐
│  Determine the initial body position │
│  for treatment according to primary  │
│         diagnosis images             │
└─────────────────────────────────┘
                 │
                 ▼
┌─────────────────────────────────┐
│   Localize the patient and align     │
│ the coordinates system before CT scan│
└─────────────────────────────────┘
                 │
                 ▼
        ┌──────────────────┐
        │     CT scan       │
        └──────────────────┘
                 │
                 ▼
        ┌──────────────────┐
        │  Draw out CT image│
        └──────────────────┘
                 │
                 ▼
    ┌──────────────────────────┐
    │ Make a primary treatment plan │
    │   according to said CT image  │
    └──────────────────────────┘
                 │
                 ▼
    ┌──────────────────────────┐
    │ Localize the patient and align│
    │    the coordinates system     │
    │        before treatment       │
    └──────────────────────────┘
                 │
                 ▼
    ┌──────────────────────────┐
    │ Carry out the registration and│
    │ fusion of B-mode ultrasound   │
    │    image and CT image         │
    └──────────────────────────┘
                 │
                 ▼
        ┌──────────────────┐
        │ Determine the final│
        │   treatment plan   │
        └──────────────────┘
                 │
                 ▼
┌──────────────────────────────────────┐
│ Carry out the 3-D treatment guided by the images│
│          registered and fused          │
└──────────────────────────────────────┘
                 │
                 ▼
        ┌──────────────────┐
        │ Analyze and report the│
        │   therapeutic effects │
        └──────────────────┘
                 │
                 ▼
        ┌──────────────────┐
        │     Finished      │
        └──────────────────┘
```

## FIG. 2A

FIG. 2B

Determine the initial body position
for treatment according to primary
diagnosis images

↓

MRI diagnosis scan
(including the localization before scan)

↓

CT diagnosis scan
(including the localization before scan)

↓

Draw out CT image
and MRI image

↓

Carry out the registration and
fusion of MRI image and CT image

↓

Determine the primary treatment plan
according to the fused image of CT and MRI

↓

Localize the patient and align the
coordinates system before treatment

↓

Carry out the registration and fusion of B-mode
ultrasound image and the fused image of CT and MRI

↓

Determine the final
treatment plan

↓

Carry out the 3-D treatment guided by the images
registered or fused

↓

Analyze and report the
therapeutic effects

↓

Finished

FIG. 2C

**FIG. 3A**

**FIG. 3B**

**FIG. 3C**

```
┌─────────────────────────────┐
│   Determine the body position │
│   for treatment: pre-position │
└─────────────────────────────┘
              │
              ▼
┌──────────────────────────────────┐
│ Register the coordinate system of therapy │
│ equipment and the coordinates system of   │
│ the locating means: diagnosis positioning │
└──────────────────────────────────┘
              │
              ▼
┌─────────────────────────────┐
│          Image scan          │
└─────────────────────────────┘
              │
              ▼
┌─────────────────────────────────┐
│   Draw out diagnosis image and   │
│ determine the Offset 1 according to │
│    the locating mark on the image   │
└─────────────────────────────────┘
              │
              ▼
┌─────────────────────────────┐
│       Fix the patient on the      │
│     treatment bed by the locating  │
│   means 8: diagnosis positioning   │
└─────────────────────────────┘
              │
              ▼
┌─────────────────────────────┐
│  Determine the Offset 2 of the    │
│    coordinate system of therapy    │
│   equipment and the coordinates    │
│   system of the locating means     │
└─────────────────────────────┘
              │
              ▼
┌──────────────────────────────────┐
│   Realize the registration of B-mode │
│ ultrasound image and diagnosis image │
│      by the Offset 1 and Offset 2     │
└──────────────────────────────────┘
              │
              ▼
┌─────────────────────────────┐
│            Draw out          │
│     the registered image     │
└─────────────────────────────┘
```

FIG. 4

FIG. 5

**REFERENCES CITED IN THE DESCRIPTION**

**Patent documents cited in the description**

- CN 98100283
- US 4932414 A